# EUROPÄISCHE PATENTANMELDUNG

(11) **EP 0 589 168 A2**
(43) Veröffentlichungstag der Anmeldung: **30.03.1994**
(21) Anmeldenummer: 93111630.5
(22) Anmeldetag: 21.07.1993
(51) Int. Cl.: C07C 29/149, C07C 31/125

(54) **Verfahren zur katalytischen Direkthydrierung von Neocarbonsäuren zu den entsprechenden Neoalkoholen**

(30) Priorität: 12.09.1992 DE 4230565
(71) Anmelder: HÜLS AKTIENGESELLSCHAFT, D-45764 Marl (DE)
(72) Erfinder: Neumann, Manfred, Dr., D-45770 Marl (DE); Voges, Heinz-Werner, Dr., D-46284 Dorsten (DE); Stehr, Michael, Dr., D-45881 Gelsenkirchen (DE)

(57) **Zusammenfassung**

Die Erfindung betrifft ein Verfahren zur katalytischen Direkthydrierung von Neocarbonsäuren mit 5 bis 13 C-Atomen zu den entsprechenden Neoalkoholen der allgemeinen Formel 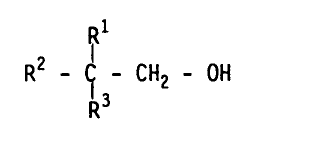
worin R₁, R₂ und R₃ gleich oder verschieden sind und lineare oder verzweigte Alkylgruppen mit 1 bis 9 C-Atomen oder Cycloalkylgruppen mit 6 bis 9 C-Atomen bedeuten, welches dadurch gekennzeichnet ist, daß die Hydrierung an auf Träger aufgebrachten Re/Pd-Metall-haltigen Katalysatoren bei Temperaturen von 150 bis 300 °C und Drücken von 50 bis 350 bar bei einer LHSV von 0,05 bis 0,5 h⁻¹ erfolgt.

## Beschreibung

Die Erfindung betrifft ein Verfahren zur Herstellung von Neoalkoholen durch katalytische Hydrierung der entsprechenden Neosäuren ohne vorhergehende Veresterung.

Unter dem Begriff Neoalkohole sind solche Verbindungen zu verstehen, bei denen das zur Hydroxylgruppe β-ständige C-Atom keine Wasserstoffatome, sondern ausschließlich Alkylgruppen trägt. Neoalkohole besitzen daher folgende allgemeine Formel: 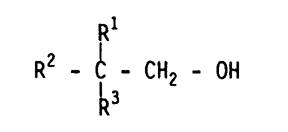
R¹, R² und R³ können verschieden oder gleich sein und sind lineare oder verzweigte Alkylgruppen oder Cycloalkylgruppen.

Neoalkohole mit 5 bis 13 C-Atomen, z. B. Neononanol, werden u. a. mitCarbonsäuren zu Estern umgesetzt, die als Weichmacher oder hochtemperaturbeständige Schmiermittel Anwendung finden. Die gute Hochtemperaturbeständigkeit der Ester, in deren Alkoholteil eine Neostruktur enthalten ist, kann auf folgende Ursache zurückgeführt werden: Die Eliminierung der Carbonsäure läuft aufgrund der fehlenden β-ständigen H-Atome im Alkoholteil des Esters nicht ab; dies ist eine Abbaureaktion, die bei hohen Temperaturen maßgeblichen Einfluß auf die Stabilität von Estern mit β-ständigen Protonen im Alkoholteil hat. Aus diesem Grund besteht ein Interesse an einem einfachen Zugang zu Neoalkoholen.

Ausgangsstoffe für die Hydrierung sind Neosäuren, die z. B. durch Koch-Synthese, d. h. Hydrocarboxylierung von verzweigten Alkenen, herstellbar sind. Das Reaktionsprodukt der Koch-Synthese mit 3-Methylhepten-2 oder 3-Methylhepten-3 als Edukt, die 2-Ethyl-2-methyl-hexansäure, sei hier beispielhaft erwähnt.

Ein weit verbreitetes Verfahren zur Synthese von Alkoholen aus den entsprechenden Säuren besteht darin, Carbonsäuren mit Alkoholen zunächst in die entsprechenden Ester zu überführen und diese Ester dann katalytisch zum Alkohol zu hydrieren. Als Katalysatoren für die Esterhydrierung haben sich insbesondere Kupferchromite mit Zusätzen von basischen Erdalkalioxiden bewährt. Diese Verfahrensweise hat den Nachteil, daß ein zusätzlicher Reaktionsschritt, d. h. die Veresterung, durchgeführt werden muß. Weiterhin muß der Ester frei von Säurespuren sein, da ansonsten Kupferchromite, die säureempfindlich sind, nur kurze Standzeiten haben.

Die Direkthydrierung von Carbonsäuren ist in einer Vielzahl von Patenten und Literaturstellen beschrieben. In EP-A 0 180 210 wird die Reduktion von Neosäuren erwähnt. Verwendet wird ein CuO/ZnO-Katalysator auf einem Al₂O₃-Träger sowie ein Nickelkatalysator auf Al₂O₃. Insbesondere wird dort auf die Reduktion von Pivalinsäure zum Neopentylalkohol abgehoben. Es zeigt sich jedoch, daß bei der Reduktion von Neosäuren mit 8 oder mehr C-Atomen Temperaturen von über 300 °C angewendet werden müssen und die Umsätze bzw. Selektivitäten mit den dort genannten Katalysatoren unbefriedigend sind. Dies ist u. a. auf die Bildung von Alkanen durch Überreduktion zurückzuführen. In der Praxis zeigt sich, daß der hier benutzte Al₂O₃-Träger für die Reduktion von Säuren bei den dafür notwendigen Temperaturen von 150 bis 300 °C nicht über einen genügend langen Zeitraum beständig ist.

Die Verwendung eines Re/Pd-Kontaktes zur Ethanol- bzw. Propanolsynthese ist in EP-A 0 198 681 beschrieben. Die Reaktion muß jedoch, um Esterbildung zu vermeiden, bei mittleren Umsatzgraden abgebrochen werden, so daß eine nachträgliche destillative Trennung des Produkts von der Eduktsäure notwendig ist. Die Hydrierung höherer Säuren bzw. Neosäuren ist nicht beschrieben.

In US-PS 4 104 478 wird die Reduktion von C₄- bis C₂₄-Fettsäuren an Re/Pd-Katalysatoren beschrieben. In α-Position zweifach alkylierte Carbonsäuren kommen nicht zum Einsatz.

In DE-PS 26 05 107 wird die Verwendung eines Re/Pd-Kontaktes zur Direkthydrierung von Dicarbonsäuren beschrieben. Insbesondere wird auf die Reduktion von Adipinsäure in wäßriger Lösung hingewiesen. Monocarbonsäuren mit Neostruktur werden nicht erwähnt.

In EP-A 0 198 682 wird an einem Pd/W- bzw. Pd/Mo-Kontakt die Reduktion von n-C₂ bis C₁₂-Carbonsäuren beschrieben. Das Reaktionsprodukt besteht jedoch zu einem großen Teil aus den entsprechenden Estern, die in einem zweiten Schritt zum Alkohol reduziert werden müssen.

Weiterhin ist die Verwendung von Rhenium oder rheniumhaltigen Katalysatoren zur Reduktion von Carbonsäuren oder Carbonsäurederivaten zu den entsprechenden Alkoholen, die in der α-Position nicht zweifach alkyliert sind, in US-PS 4 448 987, US-PS 4 338 221, US-PS 4 340 546, US-PS 4 409 148, US-PS 4 410 460, US-PS 4 324 225, EP-A 0 028 422, EP-A 0 147 219 und GB-PS 1 551 741 beschrieben.

Es wurde nun gefunden, daß sich die sterisch gehinderten Neosäuren mit besonders hohen Umsätzen und Selektivitäten an einem Re/Pd-Kontakt zu den entsprechenden Neoalkoholen umsetzen lassen. Dies ist überraschend und unerwartet, da - wie durch Beispiele belegt - geradkettige Carbonsäuren und noch verstärkter verzweigtkettige Carbonsäuren ohne Neostruktur ein deutlich schlechteres Ergebnis zeigen.

Ausgangsverbindungen sind die entsprechenden Neosäuren, die durch einen katalytischen Hydrierprozeß an einem rheniumhaltigen Katalysator mit Hilfe von Wasserstoff in die Neoalkohole umgewandelt werden.

Gegenstand der Erfindung ist ein Verfahren zur katalytischen Direkthydrierung von Neocarbonsäuren mit 5 bis 13 C-Atomen zu den entsprechenden Neoalkoholen der allgemeinen Formel 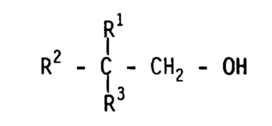
worin R₁, R₂ und R₃ gleich oder verschieden sind und lineare oder verzweigte Alkylgruppen mit 1 bis 9 C-Atomen oder Cycloalkylgruppen mit 6 bis 9 C-Atomen bedeuten, welches dadurch gekennzeichnet ist, daß die Hydrierung an auf Träger aufgebrachten Re/Pd-Metall-haltigen Katalysatoren bei Temperaturen von 150 bis 300 °C und Drücken von 50 bis 350 bar bei einer LHSV von 0,05 bis 0,5 h⁻¹ erfolgt.

Als Edukte kommen z. B. folgende Verbindungen in Betracht: 2,2-Dimethylpropionsäure (Pivalinsäure), 2,2-Dimethylbuttersäure, 2-Ethyl-2-methylbuttersäure, 2-Ethyl-2-isopropylbuttersäure, 2,2-Dimethylpentansäure, 2-Ethyl-2-methylpentansäure, 2-Ethyl-2,4-dimethylpentansäure, 2,2-Dimethylhexansäure, 2,2,5-Trimethylhexansäure, 2,2-Dimethylheptansäure, 2-Methyl-2-n-propylheptansäure, 2-Ethyl-2-methyl-hexansäure, 2,2-Diethylpentansäure, 2,2-Dimethyloctansäure, 2-Ethyl-2-methylheptansäure, 2,2-Dimethylnonansäure, 2,2-Dimethyldecansäure und 2,2-Dimethylundecansäure. Bevorzugt werden Neononan- und Neotridecansäure als Edukte für die Hydrierung eingesetzt. Besonders bevorzugt wird 2-Ethyl-2-methylhexansäure eingesetzt.

Es ist prinzipiell möglich, die Ester aus den o. a. Neosäuren mit kurzkettigen Alkoholen einzusetzen. Dies ist jedoch aus zweierlei Gründen nicht von Vorteil. Zum einen lassen sich die Ester im Vergleich zu den Säuren an rheniumhaltigen Katalysatoren weit schlechter umsetzen, und zum anderen sind die Ester von Neosäuren aufgrund sterischer Hinderung im Säuremolekül synthetisch nur schwierig zugänglich.

Die Säuren können sowohl unverdünnt als auch als Lösung in einem Lösungsmittel der Hydrierung zugeführt werden. Als Lösungsmittel kommen alle gebräuchlichen Substanzen in Betracht. Dies sind z. B. Wasser, Alkohole, Ether sowie aromatische und aliphatische Kohlenwasserstoffe. Bevorzugt werden solche Lösungsmittel, die sowohl vor als auch nach der Hydrierung homogene einphasige Gemische ermöglichen. Aufgrund der Löslichkeit der Neosäuren bzw. -alkohole und des bei der Reaktion entstehenden Wassers werden besonders bevorzugt folgende Lösungsmittel eingesetzt:
Niedere Mono- und Dialkohole, wie z. B. Methanol, Ethanol, Isopropanol oder Ethylenglykol sowie cyclische Ether, wie 1,4-Dioxan und Tetrahydrofuran. Weiterhin eignen sich auch Polyalkylenglykole bis zu einem Ethylenoxidgehalt von sechs Einheiten sowie deren ein- oder beidseitige alkylverschlossene Ether (Glyme). 1,4-Dioxan hat sich als besonders gut geeignet erwiesen.

Die Konzentration der Säure im Lösungsmittel ist in weiten Bereichen variierbar. Es ist jedoch leicht einzusehen, daß das bei der Hydrierung entstehende Wasser nur in begrenztem Maße im Hydrieraustrag löslich ist. Zur Vermeidung eines zweiphasigen Gemisches wird die Konzentration der Säure in der Eduktlösung daher bevorzugt bei 30 bis 50 Gew.-% gewählt.

Der Metallgehalt auf dem Träger liegt im Bereich von 0,1 bis 30 Gew.-%. Vor dem Hintergrund, daß durch Anheben der Konzentration an Pd über 3 Gew.-% und an Re über 5 Gew.-%, bezogen auf den Gesamtkatalysator, keine Verbesserung der Hydriereigenschaften mehr zu beobachten ist, werden die Katalysatoren maximal nur bis zu den genannten Metallgehalten hergestellt. Besonders bevorzugt sind Re-Gehalte von 0,8 bis 5 Gew.-% und Pd-Gehalte von 0,1 bis 0,7 Gew.-%.

Das Verhältnis Re/Pd kann in weiten Bereichen verändert werden. Ausgehend von Rhenium kann man das Verhältnis Re/Pd bis zu 0,2 wählen. Noch niedrigere Gehalte an Rhenium führen zu unbefriedigenden Umsätzen. Ein reiner Pd-Kontakt ist katalytisch nur unmerklich wirksam. Ein besonders bevorzugtes Verhältnis von Re/Pd liegt im Bereich von 1 bis 30.

Es ist prinzipiell möglich, die Reaktion in Anwesenheit der reinen Metalle im Autoklaven durchzuführen. Im Interesse einer technischen Anwendung jedoch muß ein kontinuierliches Verfahren gewählt werden. Hierbei erwies sich die Flüssigphasenhydrierung an einem Festbettkontakt als besonders vorteilhaft.
Die Edelmetalle sind auf einen geeigneten Träger aufgebracht. Man kann nur solche Träger verwenden, die sich unter den sauren Reaktionsbedingungen als genügend stabil erweisen. Kieselgel, Kieselgur, SiO₂/Al₂O₃ und Kohle zeichnen sich hierfür als geeignet aus. Dem Fachmann ist klar, daß auch andere Träger eingesetzt werden können. Aufgrund der Säuren kann es jedoch über kurz oder lang zu Stabilitätsverlusten an der Trägerstruktur kommen, die u. a. zu vermehrtem Austrag an Edelmetallen führen, so daß technisch akzeptable Standzeiten nicht mehr erreicht werden können. Aktivkohle erwies sich als besonders stabil gegenüber den in dieser Erfindung eingesetzten Carbonsäuren. Deshalb wird A-Kohle als Trägermaterial bevorzugt.

Die hydrieraktiven Metalle werden in Form ihrer Salze als Lösung auf den Träger gebracht und anschließend in einer reduzierenden Wasserstoffatmosphäre bei 150 bis 450 °C in die metallische Form überführt. Das Aufbringen kann durch Aufsprühen bzw. Tauchung erfolgen. Bevorzugt wird die Tauchimprägnierung. Die Metallsalze werden in Wasser gelöst, mit dem Träger in Berührung gebracht und anschließend das Lösungsmittel bei 100 bis 130 °C im leichten Vakuum abdestilliert. Als Salze für die Metalle kommen alle in Wasser leicht löslichen Verbindungen in Betracht - z. B. die Carboxylate und Nitrate sowie evtl. die Chloride, sofern in Wasser löslich. Als Rheniumverbindungen werden Ammoniumperrhenat bzw. Rheniumoxid bevorzugt.

Die Bedingungen des hier beschriebenen Verfahrens sind derartig gewählt, daß bei einem hohen Umsatz an Neosäure die Menge an durch Überhydrierung gebildetem Alkan möglichst niedrig gehalten wird. Die Selektivitäten erreichen 97 %. Die Reaktionstemperaturen für die Hydrierung liegen zwischen 150 und 300 °C, der H₂-Druck zwischen 50 und 350 bar. Bevorzugt werden Temperaturen von 190 bis 250 °C und Drücke von 250 bis 300 bar. Die Neosäurebelastung (LHSV = 1 Neosäure/1 Kontakt · h) des Katalysators liegt zwischen 0,05 und 0,5 h⁻¹. Bevorzugt werden Werte zwischen 0,1 und 0,25 h⁻¹.

Der Wassergehalt im Reaktionsaustrag bei einem Neoalkoholgehalt von ca. 40 Gew.-% sollte 10 Gew.-% nicht überschreiten, da bei höheren Wassergehalten Selektivität und Umsatz deutlich zurückgehen Dies ist darauf zurückzuführen, daß durch eine zu große Menge an Wasser das Reaktionsgemisch zweiphasig wird, was sich schlecht auf das Hydrierergebnis auswirkt. So hat es sich als günstig erwiesen, den Neosäuregehalt im Lösungsmittel nicht größer als 50 Gew.-% zu wählen. Damit wird sichergestellt, daß das durch Hydrierung entstehende Wasser zu jeder Zeit vollständig im flüssigen Reaktionsmedium löslich ist.

Die Katalysatortestungen werden unter kontinuierlichen Bedingungen durchgeführt. Hierfür wird der beladene Träger in einen für 300 bar ausgelegten, rohrförmigen Hydrierofen mit einem Verhältnis von Länge zu innerem Durchmesser von 50 - 100 : 1 gegeben. Die Aktivierung erfolgt bei 280 bis 350 °C mit einem H₂/N₂-Strom. Nach 3 bis 4 h ist die Aktivierung beendet. Mit Hilfe der ESCA-Methode (Elektronenspektroskopie für die chemische Analyse) läßt sich der Reduktionsgrad der auf dem Träger niedergeschlagenen Metalle messen. Der Reduktionsgrad beträgt 95 bis 100 %, d. h. sowohl Re als auch Pd liegen nahezu ausschließlich in metallischer Form vor.

Die im folgenden aufgeführten Beispiele sollen das Verfahren der Erfindung näher erläutern.

### Herstellung der Katalysatoren A - F

500 g Aktivkohlestrangpreßlinge (WS IV AWD von Chemviron Carbon) werden an einem Rotationsverdampfer bei 20 bis 30 mbar und 80 °C unter sehr langsamer Drehung 1 h lang evakuiert. Die für die jeweiligen Metallkonzentrationen benötigten Mengen an NH₄ReO₄ und Pd (NO₃)₂, aufgelöst in 100 ml Wasser, werden portionsweise im Vakuum eingesaugt. Das Wasser wird jeweils unter Rotation im Vakuum entfernt, bis der Träger trocken im Kolben rollt. Nach vollständigem Einbringen der Re- und Pd-haltigen Lösung wird der Katalysator 16 h bei 110 °C im Trockenschrank getrocknet. Anschließend erfolgt die Reduktion bei 280 °C im drucklosen N₂/H₂-Strom (95/5) für 16 h. Daraufhin wird die Temperatur auf 350 °C gesteigert. Nach 4 h bei dieser Temperatur wird im Laufe von 4 h schrittweise auf 100 %igen H₂-Strom umgestellt und weitere 4 h bei diesen Bedingungen gehalten. Der Katalysator ist dann einsatzbereit.

| Katalysator | Re [%] | Pd [%] | Bemerkung |
|---|---|---|---|
| A | 4 | - | Vergleich |
| B | 5,0 | 0,2 | |
| C | 4,8 | 0,5 | |
| D | 4,4 | 0,8 | |
| E | 3,6 | 1,4 | |
| F | - | 5,0 | Vergleich |

Das in den folgenden Beispielen mit Neononansäure bezeichnete Edukt ist bedingt durch den Herstellungsprozeß ein Isomerengemisch. Die Hauptkomponente ist 2-Ethyl-2-methylhexansäure. Es ist sichergestellt, daß in der Neononansäure ausschlielich tertiäre Monocarbonsäuren, d. h. α,α-dialkyliert, enthalten sind. Primäre und sekundäre Monocarbonsäuren sind nicht nachweisbar.

### Beispiele 1 - 5

Neononansäure als 30 %ige Lösung in Dioxan wird am Kopf des Reaktors zusammen mit H₂ eingespeist und am Katalysator C hydriert. Nach einer Einlaufphase, in der noch Änderungen in der Produktzusammensetzung auftreten können, wird das kontinuierlich anfallende Produkt gaschromatographisch auf seine Zusammensetzung hin analysiert. Bei 300 bar und einer konstanten LHSV von 0,1 h⁻¹, bezogen auf Neononansäure, wurden Umsätze und Selektivitäten in Abhängigkeit von der Temperatur bestimmt. Die Ergebnisse sind in Tabelle 1 zusammengefaßt:

**Tabelle 1**

| Beispiel | Katalysator | Temperatur [°C] | Umsatz [%] | Selektivitäten [%] |
|---|---|---|---|---|
| 1 | C | 170 | 33 | 98 |
| 2 | C | 190 | 73 | 97 |
| 3 | C | 210 | 96 | 98 |
| 4 | C | 230 | 97 | 98 |
| 5 | C | 250 | 98 | 92 |

### Beispiele 6 - 10

Eine 40 Gew. -%ige Lösung von Neononansäure in Dioxan wird analog zu den Bedingungen in den Beispielen 1 - 5 eingesetzt. Unter Variation der Belastung werden bei 300 bar und 230 °C die in Tabelle 2 aufgeführten Ergebnisse erhalten.

**Tabelle 2**

| Beispiel | Katalys. | LHSV [h⁻¹] | Umsatz [%] | Selekt. [%] | Ausbeute [%] |
|---|---|---|---|---|---|
| 6 | D | 0,12 | 95 | 86 | 82 |
| 7 | D | 0,14 | 96 | 91 | 87 |
| 8 | D | 0,16 | 98 | 92 | 90 |
| 9 | D | 0,20 | 97 | 94 | 91 |
| 10 | D | 0,25 | 95 | 95 | 90 |

### Beispiele 11 - 15

Neononansäure wird bei 300 bar und 230 °C bei einer LHSV von 0,13 h⁻¹ in unterschiedlichen Lösungsmitteln als 30 %ige Lösung hydriert. Der Einfluß des Lösungsmittels auf Umsatz und Selektivität wird in Tabelle 3 aufgezeigt:

**Tabelle 3**

| Beispiel | Katalysator | Lösungsmittel | Umsatz [%] | Selektivitäten [%] |
|---|---|---|---|---|
| 11 | C | Dioxan | 99 | 98 |
| 12 | C | THF | 99 | 93 |
| 13 | C | Triglyme | 82 | 91 |
| 14 | C | MeOH | 85 | 94 |
| 15 | C | Dioxan/H₂O (85/15) | 93 | 97 |

### Beispiele 16 - 21

Die Katalysatoren A - F werden im Festbettreaktor bei 300 bar und 230 °C getestet. Das Edukt ist eine 40 Gew.-%ige Lösung von Neononansäure in Dioxan.

**Tabelle 4**

| Beispiel | Katalys. | Umsatz [%] | Selekt. [%] | LHSV [h⁻¹] | Bemerkung |
|---|---|---|---|---|---|
| 16 | A | 100 | 90 | 0,22 | Vergleich |
| 17 | B | 80 | 95 | 0,20 | |
| 18 | C | 97 | 98 | 0,20 | |
| 19 | D | 97 | 94 | 0,20 | |
| 20 | E | 91 | 97 | 0,19 | |
| 21 | F | 13 | 46 | 0,10 | Vergleich |

### Beispiele 22 - 26

Bei 300 bar und 230 °C wurden 30 Gew.-%ige Lösungen der in Tabelle 5 genannten Säuren für die Hydrierung an Katalysator C eingesetzt (LHSV = 0,15 h⁻¹). Die zum Vergleich aufgeführte Trimethyladipin- bzw. Laurinsäure soll verdeutlichen, daß gute bis sehr gute Selektivitäten bei guten Umsätzen nur mit Neosäuren erreicht werden können.

**Tabelle 5**

| Beispiel | Säure | Umsatz [%] | Selektivität [%] | Bemerkung |
|---|---|---|---|---|
| 22 | Pivalinsäure | 100 | 97 | |
| 23 | Neotridecarbonsäure¹⁾ | 82 | 93 | |
| 24 | Neosäure 928²⁾ | 71 | 72 | Vergleich |
| 25 | Trimethyladipinsäure³⁾ | 81 | 59 | Vergleich |
| 26 | Laurinsäure | 95 | 58 | Vergleich |

| | | | | |
|---|---|---|---|---|
| ¹⁾ Isomerengemisch, hergestellt aus Tri-n-buten | | | | |
| ²⁾ Produkt der Firma Exxon (stark verzweigtes Produkt) | | | | |
| ³⁾ Isomerengemisch aus 2,2,4- und 2,4,4-Trimethyladipinsäure | | | | |

### Beispiele 27 - 28

Bei 300 bar und 230 °C wurden 30 Gew.-%ige Lösungen der in Tabelle 6 genannten Säuren für die Hydrierung an Katalysator C eingesetzt (LHSV = 0,15 h⁻¹). Die zum Vergleich aufgeführte n-Nonan- bzw. iso-Nonansäure soll verdeutlichen, daß gute bis sehr gute Selektivitäten bei guten Umsätzen nur mit einer C₉-Carbonsäure erzielt werden können, die eine Neostruktur hat. n-Nonansäure und in noch stärkerem Maße iso-Nonansäure bringen ein deutlich schlechteres Ergebnis als neo-Nonansäure.

**Tabelle 6**

| Beispiel | Säure | Umsatz [%] | Selektivität [%] | Bemerkung |
|---|---|---|---|---|
| 27 | n-Nonansäure | 95 | 73 | Vergleich |
| 28 | iso-Nonansäure (2-Ethyl-heptansäure) | 87 | 58 | Vergleich |
| 4 | neo-Nonansäure | 97 | 98 | |

## Patentansprüche

1. Verfahren zur katalytischen Direkthydrierung von Neocarbonsäuren mit 5 bis 13 C-Atomen zu den entsprechenden Neoalkoholen der allgemeinen Formel 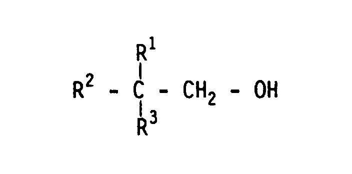 worin R₁, R₂ und R₃ gleich oder verschieden sind und lineare oder verzweigte Alkylgruppen mit 1 bis 9 C-Atomen oder Cycloalkylgruppen mit 6 bis 9 C-Atomen bedeuten,
dadurch gekennzeichnet,
daß die Hydrierung an auf Träger aufgebrachten Re/Pd-Metall-haltigen Katalysatoren bei Temperaturen von 150 bis 300 °C und Drücken von 50 bis 350 bar bei einer LHSV von 0,05 bis 0,5 h⁻¹ erfolgt.

2. Verfahren nach Anspruch 1,
dadurch gekennzeichnet,
daß der Träger Aktivkohle ist.

3. Verfahren nach Anspruch 1,
dadurch gekennzeichnet,
daß das Verhältnis Re/Pd von 1 : 1 bis 30 : 1 beträgt.

4. Verfahren nach Anspruch 3,
dadurch gekennzeichnet,
daß das bevorzugte Verhältnis Re/Pd 10 : 1 beträgt.

5. Verfahren nach den Ansprüchen 1 bis 4,
dadurch gekennzeichnet,
daß die Reaktion in Anwesenheit eines Lösungsmittels durchgeführt wird.

6. Verfahren nach Anspruch 5,
dadurch gekennzeichnet,
daß das bevorzugte Lösungsmittel Dioxan ist.

7. Verfahren nach Anspruch 1,
dadurch gekennzeichnet,
daß besonders bevorzugt ein Gemisch isomerer Neomonocarbonsäuren eingesetzt wird.

8. Verfahren nach Anspruch 7,
dadurch gekennzeichnet,
daß in dem isomeren Neosäuregemisch als Hauptisomeres 2-Ethyl-2-methylhexansäure enthalten ist.

9. Verfahren nach Anspruch 1,
dadurch gekennzeichnet,
daß der Druck bevorzugt 250 bis 300 bar beträgt.

10. Verfahren nach Anspruch 1,
dadurch gekennzeichnet,
daß die Temperatur bevorzugt zwischen 190 und 250 °C liegt.
